# EUROPEAN PATENT SPECIFICATION

(11) **EP 2 081 589 B1**
(45) Date of publication and mention of the grant of the patent: **06.10.2010**
(21) Application number: 07822652.9
(22) Date of filing: 15.11.2007
(51) Int. Cl.: A61K 38/36, A61P 7/04

(54) **COMPLEMENTATION OF FACTOR XI DEFICIENCY BY FACTOR V MUTANTS**
KOMPLEMENTIERUNG VON FAKTOR XI-MANGEL DURCH FAKTOR V-MUTANTEN
MUTANTS DU FACTEUR V POUR L'HÉMOSTASIS DANS LES CASES D'HAEMOPHILIE

(30) Priority: 16.11.2006 EP 06124261; 16.11.2006 US 859306 P; 31.07.2007 US 962842 P
(43) Date of publication of application: 29.07.2009
(73) Proprietor: Crucell Holland B.V., 2333 CN Leiden (NL)
(72) Inventor: MEIJERS, Josephus Cornelis Maria, 3737 AW Groenekan (NL); YALLOP, Christopher Adam, 2333 CN Leiden (NL); VAN DEN NIEUWENHOF, Ingrid, NL-2333 CN Leiden (NL)
(74) Representative: Manten, Annemieke
(86) International application number: PCT/EP2007/062430
(87) International publication number: WO 2008/059041

(56) References cited:
- WO-A-95/29259
- DATABASE BIOSIS [Online] BIOSCIENCES INFORMATION SERVICE, PHILADELPHIA, PA, US; 16 November 2003 (2003-11-16), MERTENS KOEN ET AL: "Factor V variants that are resistant against activated protein C may compensate for defects upstream in the coagulation cascade." XP002432590 Database accession no. PREV200400147341 cited in the application & BLOOD, vol. 102, no. 11, 16 November 2003 (2003-11-16), page 550a, 45TH ANNUAL MEETING OF THE AMERICAN SOCIETY OF HEMATOLOGY; SAN DIEGO, CA, USA; DECEMBER 06-09, 2003 ISSN: 0006-4971
- BOS M H A ET AL: "Does activated protein C-resistant factor V contribute to thrombin generation in hemophilic plasma?" JOURNAL OF THROMBOSIS AND HAEMOSTASIS : JTH MAR 2005, vol. 3, no. 3, March 2005 (2005-03), pages 522-530, XP008078605 ISSN: 1538-7933 cited in the application
- DATABASE MEDLINE [Online] US NATIONAL LIBRARY OF MEDICINE (NLM), BETHESDA, MD, US; January 2004 (2004-01), STASKO J ET AL: "[Thrombin activatable fibrinolysis inhibitor (TAFI) and its importance in the regulation of fibrinolysis]" XP002506511 Database accession no. NLM15015228 & VNITRNÍ LÉKARSTVÍ JAN 2004, vol. 50, no. 1, January 2004 (2004-01), pages 36-44, ISSN: 0042-773X

## Description

The invention relates to the field of pharmaceutical products, in particular blood clotting factors and use thereof for hemostasis.

### BACKGROUND OF THE INVENTION

Blood coagulation is a highly regulated process required to prevent blood loss in response to vascular injury. It should be triggered immediately upon injury and switched off as soon as the vasculature is intact. When this balance between activation (coagulation) and inactivation (anti-coagulation) is disturbed, a bleeding disorder or thrombotic disease may ensue. A typical example of a bleeding disorder is hemophilia.

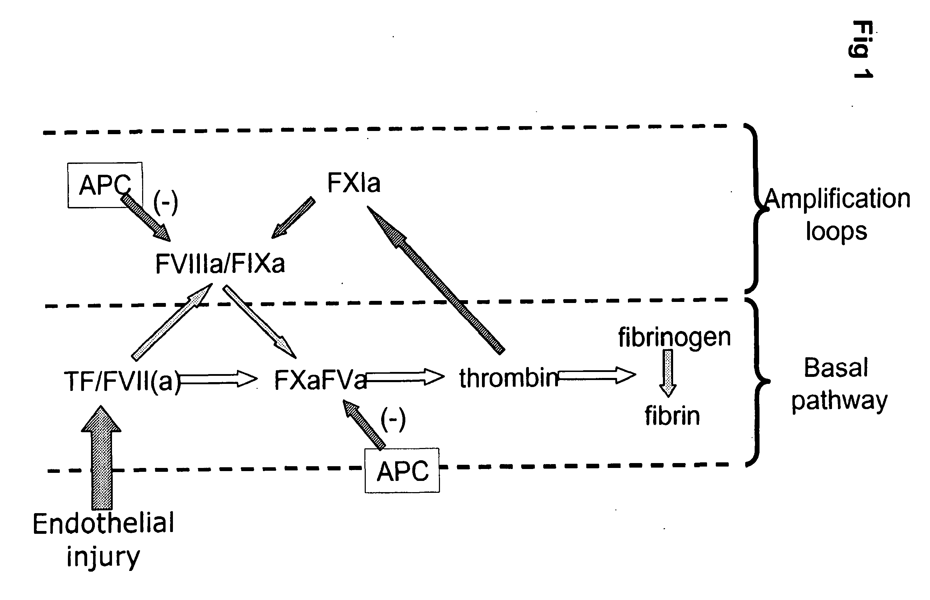

A simplified view of the coagulation system is shown in Fig. 1. Activation of the coagulation system is initiated by the formation of the TF-FVIIa complex and propagated by the action of the FVIIIa-FIXa complex. TF-FVIIa complex activates FX as well as FIX to generate FXa and FIXa, respectively. The FVIIIa-FIXa complex, similarly as the TF-FVIIa, also activates FX. Thus by activating FIX the action of TF-FVIIa on FX is amplified (Fig. 1). Under physiological conditions most hemostatic responses need this FIX- and FVIII-dependent amplification to ensure sufficient activation of FX (and hence thrombin generation). Lack of this amplification loop manifests itself in the bleeding disorders hemophilia A (FVIII deficiency) or B (FIX deficiency).

Hemophilia is typically managed by replacement therapy, which is based on the complementation of the patient's defective coagulation system with the deficient coagulation factor. Thus, hemophilia A and B patients are infused with Factor VIII and Factor IX concentrates to treat or to prevent bleeding episodes.

FV plays a central role in the coagulation cascade. Upon activation, FVa acts as a cofactor for FXa, and increases the rate of FXa-induced thrombin generation by 300,000 times compared to FXa alone (Mann and Kalafatis 2003). Factor V (FV) in its activated form thus has a critical procoagulant function.

An anti-coagulant system regulates the pro-coagulant functions of the clotting cascade. This anti-coagulant system involves activated protein C (APC), which inactivates FVIII and FV. Overall, APC constitutes a major anticoagulant protein with a significant impact on regulating the clotting system. FV also has anticoagulant effects since it can act as a cofactor for APC to assist in inactivating FVIIIa (Thorelli et al, 1999; for review see Mann et al, 2003).

APC-resistant FV mutants have been described including FV-Leiden (Arg506Gln), FV-Cambridge (Arg306Thr) and FV- Hong Kong (Arg306Gly) (Bertina et al, 1994, Svensson et al 1994, Williamson et al, 1998 and Chan, 1998). These mutants are inactivated more slowly by APC and hence prolong the activity of factor Xa. Thus, via their effect on FXa these FV mutants enhance thrombin formation. APC-resistant FV cannot act as a cofactor for APC and thus lacks the anti-coagulant effect of its wild type counterpart.

Recently, APC-resistant recombinant FV has been suggested as a therapeutic option to increase thrombin generation in hemophilia A or B patients (EP 0756638 B1; Van 't Veer et al, 1997; Bos et al, 2005).

Factor XI deficieney leads to impairment of an amplification loop in the blood coagulation cascade, resulting in Hemophilia C, which is a mild bleeding disorder and bleeding is typically induced by surgery or trauma (reviewed in O'connell, 2004; Bolton-Maggs, 2000). Hemophilia C patients can be treated with preparations containing Factor XI, such as fresh frozen plasma or FXI concentrates (see e.g., Bolton-Maggs, 2000). A replacement factor for Factor XI which is virally inactivated is not available in the US (Aledort et al, 2005). It has also been proposed to administer recombinant Factor XI for hemostatic treatment, see e.g. WO 2005/049070. Patients with severe Factor XI deficiency may develop inhibitors to Factor XI, so that treatment with Factor XI becomes ineffective (see e.g., Salomon et al, 2006). Recombinant activated Factor VII (rFVIIa) has also been used to treat patients with Factor XI deficiency (see e.g. O'Connell, 2004; Salomon et al, 2006; Shulman and Németh, 2006). However, one potential drawback of using rFVIIa is the risk for thromboembolic events when used to treat a relatively milder coagulation deficiency such as FXI deficiency (Boggio 2005). An additional disadvantage of rFVIIa is that it is commonly used in combination with an antifibrinolytic such as Traxenamic acid (O'Connell 2004). The reason for this is that Factor XI is thought to play its role in coagulation through the generation of thrombin (as does rFVIIa) but also through the inhibition of fibrinolysis by activation of TAFI (which rFVIIa cannot do).

There remains a need for further therapies to prevent or treat bleeding in subjects with Factor XI deficiency.

### BRIEF SUMMARY OF THE INTENTION

The present invention discloses the surprising finding that APC-resistant Factor V can bypass a Factor XI deficiency and restore clotting in plasma that is deficient in Factor XI. Wild-type FV cannot bypass the requirement for FXI in such plasma. It is shown that APC-resistant Factor V can restore clotting (measured by fibrin formation) in FXI-deficient plasma in the absence of added activated protein C (APC) or thrombomodulin. It is further demonstrated that the potency of APC-resistant FV to bypass FXI requirement is increased in the presence of elevated APC concentrations. These findings imply that APC-resistant FV can be used to treat and/or prevent bleeding in hemophilia C patients.

The invention provides a method for preventing or treating bleeding in a patient with a FXI-deficiency (e.g., hemophilia C), comprising administering to said patient APC-resistant FV. It is also an aspect of the invention to provide a method for reducing or preventing the possibility of generating inhibitors to Factor XI in a hemophilia C patient, comprising administering APC-resistant Factor V to the patient.

According to the aspects of the invention described above, a hemostatic amount of APC-resistant Factor V is administered to said patient. Said amount preferably is an effective hemostatic amount. The invention thus also provides a method for treatment or prophylaxis of a patient having a FXI deficiency or inhibitor, comprising administering to the patient an effective hemostatic amount of APC-resistant Factor V.

In certain embodiments, APC-resistant Factor V is administered to obtain a plasma concentration of about between 0.1 and 5, e.g. of about between 0.5 and 2 Units/ml.

In certain embodiments, the APC-resistant Factor V has a mutation of Arg306. Arg506 or both Arg 306 and Arg506 as compared to the wild type Factor V sequence (SEQ. ID. NO. 1). In one embodiment, the APC-resistant Factor V has a mutation of both Arg306 and Arg506 as compared to the wild type Factor V sequence (SEQ. ID. NO. 1).

In certain embodiments, the APC-rcsistant Factor V is free from other clotting factors.

It is also an aspect of the invention to provide the use of APC-resistant Factor V for the manufacture of a medicament for treatment or prevention (or at least reduction) of bleeding in a FXI-deficient (hemophilia C) patient.

### BRIEF DESCRIPTION OF THE FIGURES

FIG. 1. Simplified scheme of coagulation. Upon endothelial injury tissue factor (TF) becomes exposed to blood. FVII interacts with TF and becomes activated to activate in its turn FX. FXa in presence of its cofactor FVa converts prothrombin into thrombin, which in its turn generates fibrin. The system is amplified by 2 loops, one involving FVIII and FIX, and the other FXI. Activated protein C (APC) acts as an anticoagulant by inactivating FVa and FVIIIa.

FIG. 2. Scheme of assay for cofactor activity of Factor V preparations using a Chromogenic assay.

FIG. 3. Scheme of assay for clot activity of Factor V preparations, using a Prothrombin Time (PT) assay in Factor V-deficient human plasma.

FIG. 4. Scheme of assay for APC-resistance of Factor V preparations, using an APTT assay in Factor V-deficient human plasma with and without Activated Protein C (APC).

FIG. 5. Outline of Fibrin Generation Time (FGT) assay used to determine the ability of APC-resistant Factor V preparations to restore clotting in FVIII-deficient human plasma. The end point of the assay is Fibrin formation, which is recorded over time by measurement at an optical density of 405nm. The FGT (T_{1/2}max) is then calculated. Using this assay, the effect of addition of APC-resistant FV can be tested and compared to addition of FXI.

FIG. 6. FV-L/C restores clotting in FXI-depleted human plasma in the absence of added A PC. Conditions: TF dilution 1:132,000, no APC added.

Fig 7. Potency of FV-L/C in FXI-depleted human plasma is increased compared to pFXI in the presence of APC. Conditions: TF dilution 1:132,000, 9 nM APC.

### DETAILED DESCRIPTION OF THE INVENTION

Hemostasis refers to the processes, such as coagulation activation, involved in stopping bleeding. Accordingly, a "hemostatic amount" as used herein is thus defined as an amount (of a clotting factor, e.g. APC-resistant FV) sufficient to restore thrombin generation or fibrin formation up to levels sufficient to support coagulation necessary for stopping or preventing bleeding. This can for instance normally be reached by adding the amount of the lacking clotting factor (e.g. FXI in hemophilia C plasma) which would normally prevent and/or stop bleeding. While there is no direct correlation to the levels of Factor XI and the bleeding severity (many partially deficient patients have a severe bleeding tendency while some severe deficient patients show only a mild bleeding tendency), severe FXI deficient patients are typically characterized by circulating FXI levels of less than 0.1 U/ml plasma, while partial FXI deficiency is characterized by FXI levels of 0.1-0.6 U/ml plasma (for review see Bolton-Maggs, 2000). Levels in normal individuals range from 0.6-1.39 U/ml, the mean level being around 1 U/ml. Current treatments of bleeding in FXI deficient patients that receive either a FXI concentrate or Fresh Frozen Plasma (FFP) aim at reaching plasma levels of more than 0.2 U/ml FXI. Using the in-vitro data to determine relative potency of FV-L/C to FXI, this would equate to achieving a maximum of 3 U/ml APC-resistant FV (equivalent to 0.3 U/ml FXI in absence of APC) to a minimum of 0.3 U/ml APC-resistant FV (equivalent to 0.3 U/ml FXI in presence of APC).

The present invention discloses that hemostatic amounts can be reached with APC-resistant Factor V in FXI-depleted plasma as well, e.g. by addition of this molecule to reach concentrations of between about 0.1 and 5 U/ml plasma. In certain embodiments, said hemostatic amounts in FXI-depleted plasma are obtained by concentrations of about between 0.5 and 2 U/ml plasma, e.g. at about 1 Units/ml plasma. Thus, the invention provides a method for prevention or treatment of bleeding in a patient with a FXI-deficiency (hemophilia C), comprising administering to said patient APC-resistant FV.

One unit of a blood clotting factor in general is defined as the amount that is present in 1 ml pooled normal human plasma. One unit of Factor V activity or antigen corresponds to the amount of Factor V in 1 ml of normal plasma, which is about 5-10 µg/ml. For APC-resistant Factor V, one unit is thus defined as having the same amount of Factor V antigen as present in pooled human plasma. Accordingly, 1 U of FV-L/C corresponds to about 5-10 µg/ml.

The present invention surprisingly discloses that APC-resistant Factor V can restore fibrin generation in plasma that is deficient in Factor XI, and is more potent in such plasma under conditions with high APC levels. The required level of APC-resistant FV (e.g. 0.1-5 U/ml plasma) in a human patient with a deficiency in Factor XI can be obtained by administration of APC-resistant FV at a frequency and dosage (per kg of body weight) that will be dependent on pharmacokinetics, in vivo recovery and potency of the APC-resistant FV preparation, as is well known and can be routinely determined by the skilled person. It is therefore within the skill of the artisan to determine the dose and frequency to obtain the desired levels of APC-resistant Factor V in the plasma of the subject. The plasma volume is typically about 50 ml per kg body weight. Thus the dose and frequency can be varied by the clinician to arrive at the optimum therapy. According to the present invention, generally a dose of about from 0.1 to 5, e.g. about from 0.5 to 2 Units/ml plasma can be used. The frequency of dosing will ordinarily be every 1 to 7 days for prophylaxis. In certain embodiments of the present invention, APC-resistant FV may be administered at 0.1-500 Units per kg body weight, e.g. between 1-50 U/kg.

In certain embodiments, the subject having a Factor XI deficiency is a patient suffering from hemophilia C. In certain embodiments, said subject has a mutation in the Factor XI gene, e.g. resulting either in homozygous type I, Type II or Type III deficiencies or heterozygous combinations of deficiency (for example Type II/Type III) or heterozygous single deficiencies (eg Type III/Normal). In certain embodiments, the subject has inhibitors to Factor XI.

The APC-resistant Factor V can be used according to the invention for prophylaxis, meaning that it is used for prevention of bleeding, i.e. at times when no bleeding occurs. It can also be used for treatment of bleeding, i.e. at times when bleeding already started, to stop the bleeding.

Bleeding in FXI deficient patients is particularly prevalent in certain soft tissues with a high fibrinolytic activity (for example urinary tract, gums, tonsils, nasal cavity etc). Human plasma from normal persons contains low but measurable amounts of activated protein C (Gruber et al, 1992). The concentration of APC depends on the levels of Thrombomodulin (TM). TM is located on the endothelium. TM concentration in the microcirculation (capillaries) has been shown to be particularly high (100-500 nmol/L; Esmon, 1989). Therefore, most thrombin in the microvascular bed will be bound to TM and activate protein C. Hence, the concentration of APC is the highest in the microcirculation. Thus, APC-resistant FV may be very suitable for treating bleeding episodes in the micmcirculation, e.g. in the joints, muscles or soft tissues.

The Factor V (FV) molecule as present in blood of normal individuals is composed of three A domains, one B domain, and two C domains. This structure resembles that of factor VIII (Jenny et al. 1987). Upon synthesis in the liver, the FV molecule undergoes multiple posttranslational alterations, including sulfation, phosphorylation and glycosylation. FV in plasma is a single-chain protein with MW 330kD. During activation by thrombin, FV undergoes several proteolytic cleavages, i.e. at Arg709, Arg1018 and Arg1545, and moreover, the large connecting B domain is released from the molecule. As a result its cofactor activity for FXa is enhanced by several orders of magnitude. The resulting FVa is composed of the noncovalently associated heavy (A1-A2) and light (A3-C1-C2) chains. By serving as an essential cofactor of FXa, FVa has a clear procoagulant effect. The activity of activated FV is tightly regulated by activated protein C (APC), which inactivates the molecule by cleavage at one or more of several residues to yield FVi (for review see Mann et al, 2003).

Human Factor V contains cleavage sites for activated protein C (APC), to be cleaved between Arg³⁰⁶-Asn³⁰⁷, Arg⁵⁰⁶-Gly⁵⁰⁷, Arg⁶⁷⁹-Lys⁶⁸⁰ and Arg¹⁷⁶⁵-Leu¹⁷⁶⁶ (EP 0756638). An "APC-resistant Factor V" molecule as used herein will result in a clotting time (e.g. in an APTT test) that is less than 150%, typically less than 120% (e.g. 80-120%, or 90-110%), of the clotting time in the absence of added APC, under conditions where APC is present at a concentration such that wt Factor V (from pooled human plasma) has a clotting time that is at least 150% (typically at least 180%, e.g. 200-30/%) of the clotting time in the absence of added APC. APC- resistant Factor V as used in the present invention preferably is a Factor V (FV) molecule having a modification at or near a cleavage site for APC so as to reduce or abolish the activity of APC to cleave at the original cleavage site, i.e. to induce APC resistance. Preferably the APC resistant FV is derived from the human FV sequence, but it could also be derived from FV from another species, e.g. monkey, bovine, porcine etc. In certain preferred embodiments the APC resistant FV is derived from human FV and has a modification at amino acid position Arg³⁰⁶ (one possible mutation is into Thr which yields a FV molecule referred to as 'Factor V-Cambridge' or 'FV-C'), or Arg⁵⁰⁶ (one possible mutation is into Gln which yields a FV molecule referred to as 'Factor V-Leiden' or 'FV-L'), or Arg⁶⁷⁹, or both Arg³⁰⁶ and Arg⁵⁰⁶ (e.g. mutations of Arg306 into Thr and Arg⁵⁰⁶ into Gln yielding a molecule also referred to as 'Factor V-Leiden/Cambridge' or 'FV-L/C'), or other combinations thereof (all as compared to the mature sequence disclosed in Jenny et al, 1987, or to the amino acid sequence as present in Swissprot entry P12259, which both represent wild type human Factor V sequences; SEQ. ID. NO. 1 in the present disclosure provides a wild-type mature human Factor V sequence). The modification in certain embodiments is an amino acid substitution. In certain embodiments, the Arginine residue that precedes the APC-cleavage site is changed into a Gln, Ile, Thr. Gly, or any other amino acid. In certain embodiments. Arg³⁰⁶ is replaced by Thr ('FV-R306T'). In other embodiments, Arg⁵⁰⁶ is replaced by Gln ('FV-R506Q'). In certain embodiments. Arg³⁰⁶ is replaced by Thr and Arg⁵⁰⁶ is replaced by Gln ('FV-R306T/R506Q'). It will be clear to the skilled person that further amino acid additions, deletions and/or substitutions could be present in the molecule without further affecting the biological activity of APC-resistant FV for the purpose of the present invention, e.g. the B-domain could be deleted (Pittman et al 1994), or allelic variants could be used, and it will be understood that such molecules are included within the definition of APC-resistant Factor V according to the present invention. Preparation of such variants can be done by routine molecular biology methods. It is preferred that APC-resistant FV is in non-activated form for use according to the present invention, but it could also be wholly or in part in its activated form (APC-resistant Factor Va) for use according to the present invention, and thus APC-resistant Factor Va is included within the scope of the term APC-resistant Factor V according to the present invention. Activation of FV during purification or storage in vitro may be prevented by the addition of thrombin inhibitors, and/or storage at pH lower than 7.4, etc. The APC-resistant FV molecules used herein will include variants, fragments, functional equivalents, derivatives, homologs and fusions of the native APC-resistant FV molecule so long as the product retains the APC resistance and Factor V procoagulant property. Useful derivatives generally have substantial sequence similarity (at the amino acid level) in regions or domains of the APC resistant FV molecules as identified above (FV-R306T, FV-F506Q, FV-R306T/R506Q), e.g. are at least 50%, at least 60%, preferably at least 70%, more preferably at least 80%, still more preferably at least 90%, still more preferably at least 95% identical in amino acid sequence with the APC-resistant Factor V molecules identified above.

Griffin et al have described stabilized Factor V molecules with engineered disulfide bonds (US 2003/0125232). Such molecules are also functionally APC-resistant (they are cleaved by APC, but because of the disulfide bridges this cleavage does not remove the procoagulant activity), and are therefore included within the scope of the term APC-resistant Factor V according to the present invention.

Preparations containing APC-resistant FV may be obtained by purification from plasma of patients that have a mutation in the FV gene leading to APC-resistance (see e.g. EP0756638), e.g. having a FV-L or FV-C mutation. Preferably however, the APC-resistant FV molecules are produced through recombinant DNA technology involving expression of the molecules in cells, preferably eukaryotic cells, e.g. Chinese hamster ovary (CHO) cells, HEK293 cells, BHK cells, PER.C6 cells (as deposited at the ECACC under no. 96022940; for recombinant expression of proteins in PER.C6 cells see e.g. US patent 6,R55,544), yeast, fungi, insect cells, and the like, or prokaryotic cells, or transgenic animals or plants. In certain embodiments, recombinant expression is achieved in PER.C6 cells that further over-express a sialyltransferase, e.g. human α-2,3-sialyltransferase under control of a heterologous promoter (see e.g. WO 2006/070011). Methods for recombinant expression of desired proteins are known in the art, and recombinant production of APC-resistant FV has been described (e.g. EP 0756638 B1; Egan et al, 1997; Bos et al, 2005). In general, the production of a recombinant protein, such as APC-resistant FV of the invention, in a host cell comprises the introduction of nucleic acid encoding the protein in expressible format into the host cell, culturing the cells under conditions conducive to expression of the nucleic acid and allowing expression of the said nucleic acid in said cells. Nucleic acid encoding a protein in expressible format may be in the form of an expression cassette, and usually requires sequences capable of bringing about expression of the nucleic acid, such as enhancer(s), promoter, polyadenylation signal, and the like. Several promoters can be used for expression of recombinant nucleic acid, and these may comprise viral, mammalian, synthetic promoters, and the like. In certain embodiments, a promoter driving the expression of the nucleic acid of interest is the CMV immediate early promoter, for instance comprising nt. -735 to +95 from the CMV immediate early gene enhancer/promoter. The nucleic acid of interest may be a genomic DNA, a cDNA, synthetic DNA, a combination of these, etc. Cell culture media are available from various vendors, and a suitable medium can be routinely chosen for a host cell to express the protein of interest, here APC-resistant Factor V. The suitable medium may or may not contain serum.

Harvesting and purification of the protein of interest can be done according to methods routinely available to the skilled person, e.g. employing chromatography such as affinity chromatography, ion-exchange chromatography, size-exclusion chromatography, and the like. Protocols for purification of APC-resistant Factor V from blood or plasma of patients with a FV-L mutation have been described (EP 0756638). Protocols for purification of APC-resistant Factor V from recombinant cell culture have also been described (e.g. Bos et al, 2005, who describe a procedure based on affinity chromatography with a monoclonal antibody) and are thus available for the skilled person.

For administering to humans, the invention may employ pharmaceutical compositions comprising the APC-resistant Factor V and a pharmaceutically acceptable carrier or excipient. In the present context, the term "Pharmaceutically acceptable" means that the carrier or excipient, at the dosages and concentrations employed, will not cause any unwanted or harmful effects in the patients to which they are administered. Such pharmaceutically acceptable carriers and excipients are well known in the art (see Remington's Pharmaceutical Sciences, 18th edition, A. R. Gennaro, Ed., Mack Publishing Company [1990]; Pharmaceutical Formulation Development of Peptides and Proteins, S. Frokjaer and L. Hovgaard, Eds., Taylor & Francis [2000]; and Handbook of Pharmaceutical Excipients, 3rd edition, A. Kibbe, Ed., Pharmaceutical Press [2000]). The APC-resistant FV of this invention preferably is formulated and administered as a sterile solution although it is within the scope of this invention to utilize lyophilized preparations. Sterile solutions are prepared by sterile filtration or by other methods known per se in the art. The solutions are then lyophilized or filled into pharmaceutical dosage containers. The pH of the solution generally is in the range of pH 3.0 to 9.5, e.g pH 5.0 to 7.5. The protein typically is in a solution having a suitable pharmaceutically acceptable buffer, and the solution of protein may also contain a salt. Optionally stabilizing agent may be present, such as albumin. In certain embodiments, detergent is added. For use in this invention APC-resistant FV may be formulated into an injectable preparation. Parenteral formulations are suitable for use in the invention, preferably for intravenous administration. These formulations contain therapeutically effective amounts of APC-resistant FV, are either sterile liquid solutions, liquid suspensions or lyophilized versions and optionally contain stabilizers or excipients.

APC-resistant FV may be administered by injection intravenously, or by other administration routes and/or sites, at a hemostatic amount, which thus is sufficient to correct FXI deficiency.

The APC-resistant Factor V administered to the patients according to the invention may be free from other blood clotting factors. It is shown herein that APC-resistant FV can be administered to restore hemostasis in FXI-depleted plasma, without addition of Factor XI. In other embodiments, the APC-resistant Factor V may be combined with other blood clotting factors, e.g. one or more of Factor VIII, Factor VIIa, Factor IX, and the like. In certain embodiments it is free of (APC-resistant and/or wild-type) Factor Va.

According to the present invention hemophilia C patients are treated with APC-resistant Factor V. In preferred aspects, Factor XI needs no longer to be administered, or administering of Factor XI can be diminished to much lower levels, for instance to levels sufficiently low to not provoke an immune response in the patient to F XI.

In certain embodiments, the invention provides a method for prevention or treatment according to the invention, wherein the hemostatic level of APC-resistant Factor V is determined in an in vitro assay comprising: a) providing plasma from a hemophilia C patient with (a dilution of) tissue factor, Ca²⁺, and optionally activated protein C or thrombomodulin at concentrations where clotting time (or fibrin/thrombin formation) is dependent from addition of Factor XI, b) measuring fibrin or thrombin generation in the absence of FXI, c) measuring fibrin or thrombin generation in the presence of a dose between 0.1 and 5 U/ml of FXI, and d) measuring fibrin or thrombin generation in the absence of FXI in the presence of APC-resistant Factor V, to determine a hemostatic level of said APC-resistant Factor V to replace the FXI that is deficient in said plasma. The invention further provides a method for testing the capacity of APC-resistant Factor V to bypass a FXI deficiency in a plasma, comprising: a) providing plasma which has a deficiency in Factor XI with (a dilution of) tissue factor, Ca²⁺, and optionally activated protein C and/or thrombomodulin at concentrations where clotting time (or fibrin/thrombin generation) is dependent from addition of FXI; b) measuring fibrin or thrombin generation in the absence of FXI; c) measuring fibrin or thrombin generation in the presence of a dose between 0.1 and 5 U/ml of FXI; and d) measuring fibrin or thrombin generation in the absence of FXI in the presence of APC-resistant Factor V. to establish the capacity of APC-resistant Factor V to replace the FXI that is deficient in said plasma. Preferably the assay is performed under conditions with APC (or thrombomodulin, which induces APC), and in certain embodiments, the effect of APC is tested at different concentrations.

The practice of the present invention will employ, unless otherwise indicated, conventional techniques of molecular biology and the like, which are within the skill of the art. Such techniques are explained fully in the literature. See e.g., Molecular Cloning: A Laboratory Manual, (J. Sarnbrook et al., Cold Spring Harbor Laboratory, Cold Spring Harbor, N.Y., 1989); Current Protocols in Molecular Biology (F. Ausubel et al., eds., 1987 updated); Essential Molecular Biology (T. Brown ed., IRL Press 1991); Gene Expression Technology (Goeddel ed., Academic Press 1991); Methods for Cloning and Analysis of Eukaryotic Genes (A. Bothwell et al. eds., Bartlett Publ. 1990); Gene Transfer and Expression (M. Kriegler, Stockton Press 1990); Recombinant DNA Methodology (R. Wu et al. eds., Academic Press 1989); PCR: A Practical Approach (M. McPherson et al., IRL Press at Oxford University Press 1991); Oligonucleotide Synthesis (M. Gait ed., 1984); Cell Culture for Biochemists (R. Adams ed., Elsevier Science Publishers 1990); Gene Transfer Vectors for Mammalian Cells (J. Miller & M. Calos eds., 1987); Mammalian Cell Biotechnology (M. Butler ed., 1991); Animal Cell Culture (J. Pollard et al. eds., Humana Press 1990); Culture of Animal Cells, 2.sup.nd Ed. (R. Freshney et al. eds., Alan R. Liss 1987); Flow Cytometry and Sorting (M. Melamed et al. eds., Wiley-Liss 1990); the series Methods in Enzymology (Academic Press, Inc.); and Animal Cell Culture (R. Freshney ed., IRL Press 1987); and Wirth M. and Hauser H. (1993) Genetic Engineering of Animal Cells, In: Biotechnology Vol. 2 Puhler A (ed.) VCH, Weinhcim 663-744.

### EXAMPLES

### Example 1: Recombinant production and testing of APC-resistant Factor V

Using routine molecular biology methods, three expression vectors were constructed, one containing the wild-type Factor V coding region, one containing a point mutation at amino acid position 506 (Arg506 to Gln, Factor V Leiden), and one containing a double mutation (Arg506 to Gln, and Arg306 to Thr). The factor V coding regions were.inserted behind a CMV promoter into expression vector pcDNA2001Neo(-), resulting in pCP-FV-wt (containing wild-type Factor V coding sequence), pCP-FV-L1 (containing the factor V coding sequence but with a mutation resulting in the R506Q mutation in the protein; Leiden mutant), and pCP-FV-LC1 (containing the factor V coding sequence but with a mutation resulting in the R506Q and the R306T mutation in the protein; Leiden/Cambridge double mutant).

The factor V sequence used (Bos et al., 2005) encoded the Factor V amino acid sequence as present in Swissprot entry P12259. Amino acid positions are according to the Factor V coding sequence, but after processing of the 28 amino acid leader peptide.

Similar expression plasmids with the same Factor V sequences, but with in addition a human α-2,3-sialyltransferase cDNA (Genbank accession number L23767, see also US patent 5,494,790) under control of a separate CMV promoter, were also constructed and used for obtaining clones expressing APC-resistant Factor V.

For the following, expression vector pCP-FV-LC1 (encoding FV-R306T/R506Q, further called FV-L/C) was used.

Stable PER.C6 cell lines expressing rFV-L/C were generated using standard molecular biology and cell culture techniques (e.g. US patent 6,855,544, WO 2006/070011). Cell lines that were transected with the expression vector containing only the Factor V-L/C cDNA were termed PER,C6-FV-L/C. Cell lines that were transfected with the expression vector containing the Factor V-L/C and the human α-2,3-sialyltransferase cDNA were termed PER.C6-FV-L/C-ST. The products produced by these cells are referred to as rEV-L/C and rFV-L/C-ST, respectively.

Cell lines were tested for production of recombinant protein by measuring FV levels in culture supernatant with an ELISA using polyclonal sheep anti-human FV IgG antibodies (sheep a-human Factor V; Kordia, Leiden, the Netherlands). Cell-lines producing the highest amounts of factor V were used for production of recombinant factor V.

Cell culture supernatants were produced from these cell lines in roller bottles in serum-containing culture media (e.g. DMEM with 2.5% FCS), using standard cell culture techniques. FV-L/C was purified using standard chromatography techniques, including immuno-affinity and ion-exchange chromatography (see e.g. Bos et al, 2005).

Purified samples were stored in a buffer containing 50 mM Tris/HCl (pH 7.4), 100 mM NaCl, 5 mM CaCl₂ and 50% glycerol (v/v), FV-L/C is stable (SDS-PAGE. Western blot, chromogenic assay) for >6 months in this formulation.

Plasma FV was obtained using the same procedure. Normal human plasma (Sanquin Plasma Products, Amsterdam, the Netherlands) was used as a source of FV,

On a 5% SDS-PAGE gel stained with silver, all FV species displayed a predominant band at 330 kDa, and a secondary band at 220 kDa. By immunoblotting using polyclonal anti-FV-IgG, the bands were identified as FV.

The activity of the preparations was tested for specific chromogenic and clot activity as well as for APC-resistance.

Chromogenic activity was tested in the following assay.

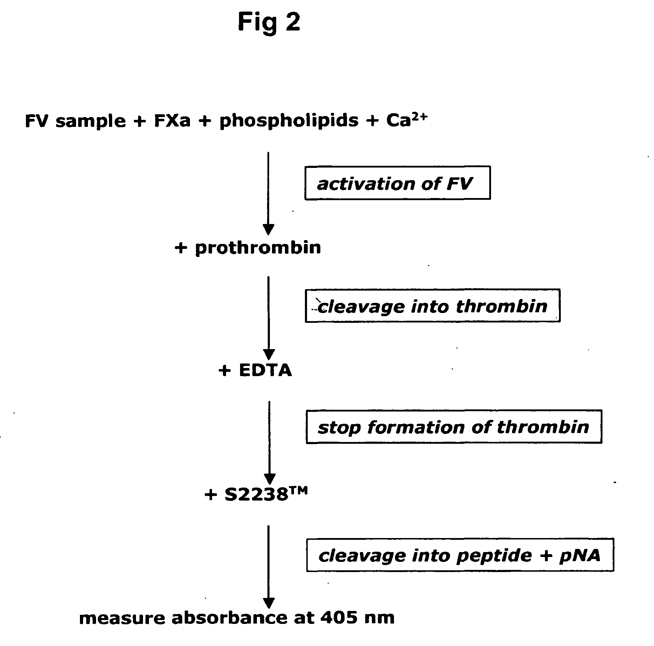

Each sample (12.5 µl ) was added to 50 µl of an activation mix containing 2 nM FXa (Kordia), 20 µM PTT reagents (Roche), CaCl₂ in a buffer containing 0.1 M NaCl, 0.05 M TRIS and 0.1% (w/v) HSA (Sigma) and 12.5 µl of Prothrombin (Kordia) and the plate incubated for 5 minutes at 37°C. The reaction was then stopped by the addition of 12.5 µl of 0.1M EDTA in 0.1 M NaCl and 0.05 M TRIS buffer. A chromogenic substrate (S2238, Chromogenix) was added (12.5 µl) and the reaction read at 405nm. Fig. 2 shows a schematic view of the chromogenic assay. Pooled plasma was used as a standard (FV concentration = 1 U/ml). The specific chromogenic activity was calculated from the chromogenic activity (U^{Chr}) divided by the Antigen concentration (U^{Ag}). All FV-L/C preparations prepared as described above showed specific chromogenic activity.

**Table 2. Activity of FV-L/C.**

| | Specific chromogenic activity (U^{Chr}/U^{Ag}) | Specific clot activity (U^{Cl}/U^{Ag}) |
|---|---|---|
| PER.C6-FV-L/C (3 batches) | 1.03 (SD^{+/-}0.23) | 1.53 (SD^{+/-}0.12) |
| PER.C6-FV-L/C-ST (4 batches) | 0.60 (SD^{+/-}0.08) | 2.23 (SD^{+/-}0.05) |
| purified plasma FV (4 batches) | 0.73 (SD^{+/-}0.21) | 0.90 (SD^{+/-}0.22) |

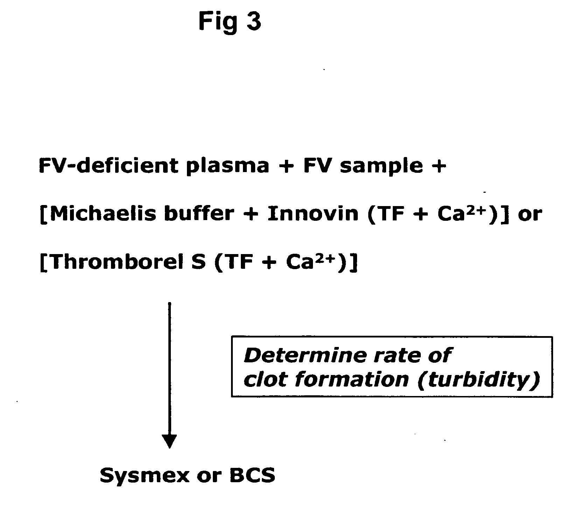

Clot activity was tested in a prothrombin time (PT) assay performed using FV-deficient human plasma. Fig. 3 shows a schematic view of the clot activity assay. Briefly, purified preparations were added to FV-deficient plasma (Dade Behring, Liederbach, Germany) employing normal human plasma as reference. Clotting was induced with Innovin^{®} (Dade Behring) or with Thromborel S (Dade Behring). Pooled plasma was again used as a standard. One unit of factor V activity or antigen is similar to the amount of FV in I mL of normal plasma (± 8 µg/mL). The specific clot activity was calculated from the clot activity (U^{Cl}) divided by the Antigen concentration (V^{Ag}). The results confirm that the produced FV-L/C has clot activity (Table 2). In fact, the somewhat higher specific clot activity of FV-L/C compared to wild type plasma derived FV may be due to the APC-resistance of FV-L/C.

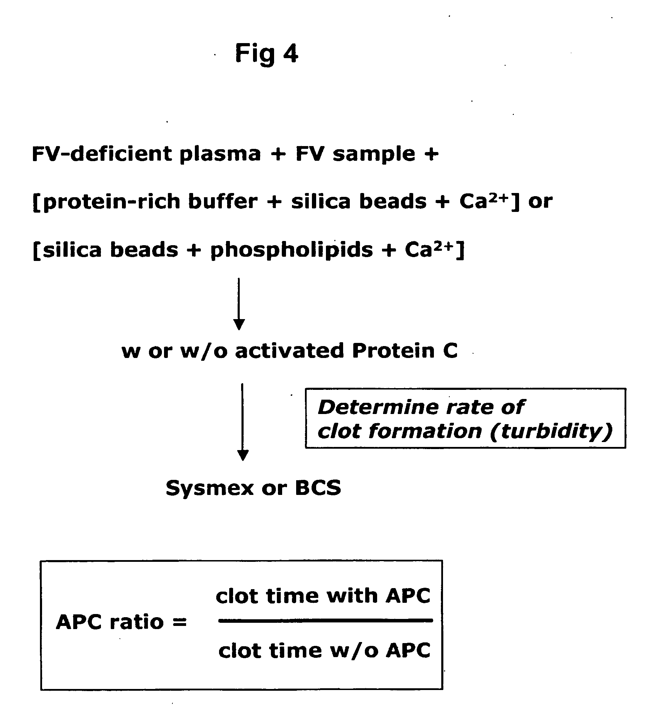

APC-resistance was tested in an Activated Partial Thromboplastin Time (APTT) assay in FV-deficient human plasma with and without APC (Kordia, Leiden, The Netherlands). Fig. 4 shows a schematic view of this assay. The results confirm that the produced FV-L/C is fully APC-resistant (Table 3).

**Table 3. APC-resistance of FV-L/C.**

| Preparation | without APC (sec.) | with APC (sec.) | APC ratio |
|---|---|---|---|
| PER.C6-FV-L/C (3 batches) | 53.3 (SD^{+/-}10.9) | 49.2 (SD^{+/-}8.2) | 0.92 (SD^{+/-}0.03) |
| PER.C6-FV-L/C-ST (4 batches) | 49.8 (SD^{+/-}8.9) | 45.3 (SD^{+/-}8.9) | 0.91 (SD^{+/-}0.02) |
| purified plasma FV (4 batches) | 61.6 (SD^{+/-}13.4) | 146.8 (SD^{+/-}21.8) | 2.39 (SD^{+/-}0.24) |

In conclusion, the biochemical characterisation of the produced FV-L/C demonstrates that we were able to obtain a preparation with a purity of over 90% at a concentration of more than 1 mg/ml, which has a specific Factor V cofactor activity, has clot activity and is fully APC-resistant.

### Example 2: FV-L/C restores clotting in FXI-depleted plasma

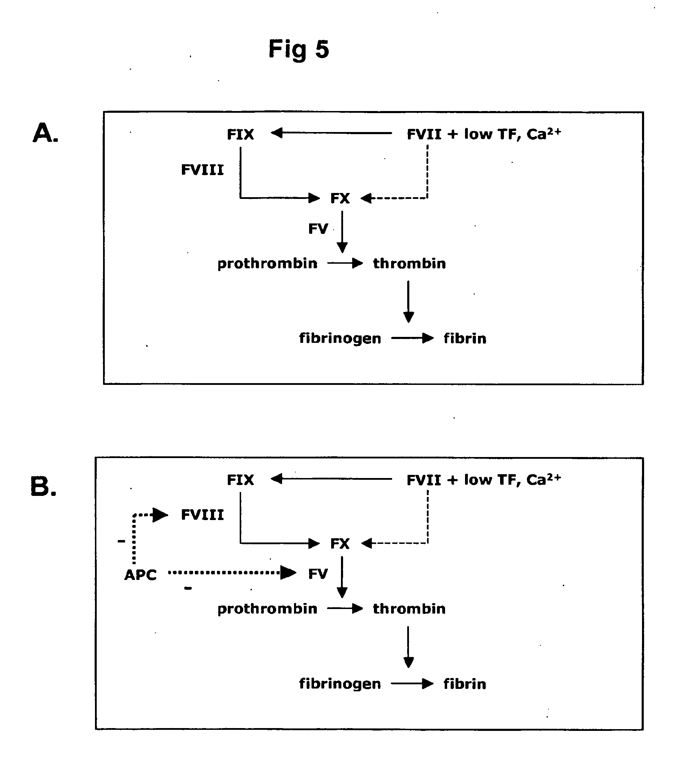

Purified rFV-L/C molecules were tested using a Fibrin Generation Time (FGT) assay (schematically shown in Fig. 5), performed in FXI immune depleted human plasma.

The assay was established using FXI-immune depleted plasma. Tissue Factor (TF) and Activated Protein C (APC) concentrations were titrated to give a dose response for Factor XI. Thrombin formation was triggered by the addition of TF in the presence of APC. The endpoint of the assay is clotting time (or fibrin generation time). TF dilution 1:132,000 (Innovin^{®}, Dade Behring, Germany) was used in the assays in the following examples.

One hundred microliters of FXI-immune depleted human plasma (Dade Behring, OSDF135) was introduced in duplicate into microtiter plates (low binding, flat bottom). Factor XI (recombinant FXI produced in BHK cells (Meijers et al, 1992)) or recombinant FV-L/C (see example 1) or purified plasma FV was added at concentrations indicated in the Figs. After addition of 75 µl of HEPES buffer (25 mM HEPES (Boehringer Mannheim), 137 mM NaCl (Merck) and 0.1% Ovalbumin (Sigma, A-5503), pH 7.4), the samples were incubated for 5 min. at 37°C. Then, 75 µl of a preheated (37 °C) dilution of TF (Innovin, Dade Behring, B4212-50) was added. Dilutions of TF were made in HEPES calcium buffer: 25 mM HEPES (Boehringer Mannheim), 137 mM NaCl (Merck), 0.1% Ovalbumin (Sigma, A-5503), 38 mM CaCl₂, pH 7.4. After mixing, the samples were immediately analyzed for fibrin generation. Fibrin generation was measured in time by use of the SpectraMax microtiterplate reader and Softmax pro software.

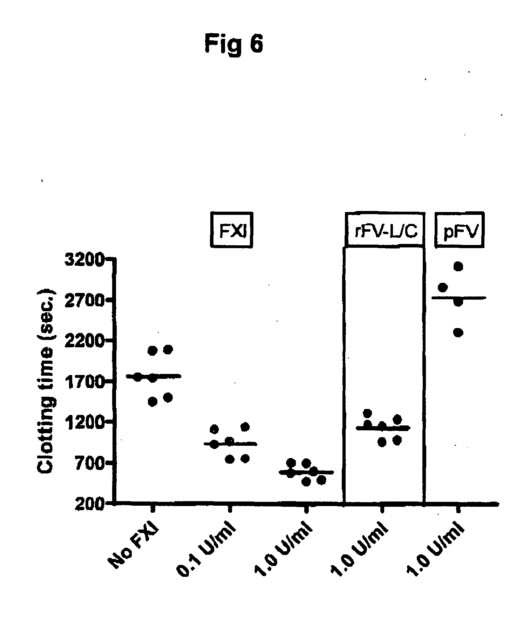

FV-L/C was able to reduce clotting time in FXI-immune depleted plasma in the absence of added APC (Fig. 6). In FXI-immune depleted human plasma, 1 U/ml rFV-L/C restores the clotting time equivalent to approximately 0.1 U/ml of the rFXI (Fig. 6). It may therefore be considered that APC-resistant Factor V is suitable for restoring or maintaining hemostasis in FXI-deficient plasma at low or absent APC levels (endogenous APC concentrations in human plasma are typically in the 60-80 pM range). Similar data were obtained using rFV-L/C-ST.

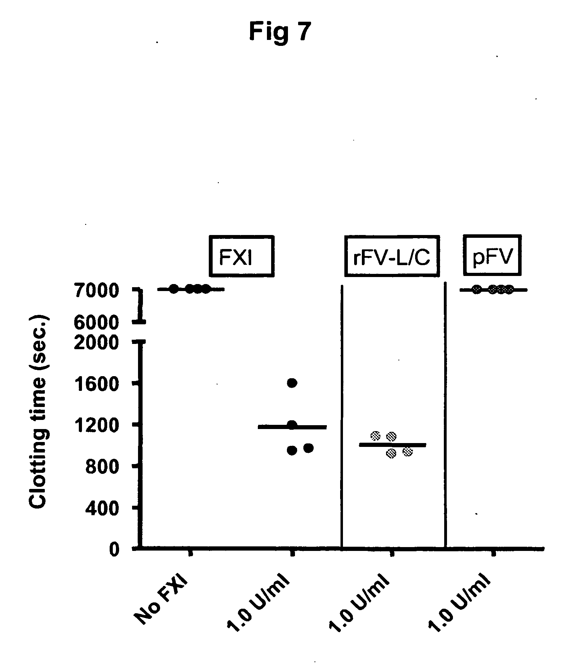

The effect of APC addition was tested, since APC plays an important role in the regulation of blood coagulation under physiological conditions. Fig 7 shows that the potency of rFV-L/C is increased in the presence of 9 nM APC when compared to pFXI (Hemoleven) in FXI-immune depleted human plasma. The addition of 1 U/ml of rFV-L/C restored the clotting time of FXI-immune depleted human plasma to a similar extent as 1 U/ml of rFXI. Similar data were obtained using rFV-L/C-ST.

Thus, these experiments highly surprisingly demonstrate that rFV-L/C can restore or maintain hemostasis in FXI-deficient plasma.

### REFERENCES

Aledort LM, Goudemand J; Hemoleven Study Group. 2005. Am J Hematol. 80: 301-302. United States' factor XI-deficiency patients need a safer treatment.
Bertina RM, Koeleman BP, Koster T, Rosendaal FR, Dirven RJ, de Ronde H, van der Velden PA, Reitsma PH. 1994 Nature 369 (6475): 14-5. Mutation in blood coagulation factor V associated with resistance to activated protein C.
Boggio P. 2005. J. Thromb. Haemostas. Suppl.1 Vol. 3.
Bolton-Maggs PH. 2000. Haemophilia 6 Suppl 1: 100-109. Factor XI deficiency and its management.
Bolton-Maggs PH. 2004. Factor XI deficiency and its management. Haemophilia 10: 593-628.
Bos MHA, Meijerman DWE, van der Zwaan C, Mertens K (2005) Does activated protein C-resistant factor V contribute to thrombin generation in hemophilic plasma? J Thromb Haemost 3: 522-530.
Chan WP, Lee CK, Kwong YL, Lam CK, Liang R. A Novel Mutation of Arg306 of Factor V Gene in Hong Kong Chinese. Blood 1998;91:1135-39
Egan JO, Kalafatis M, Mann KG. The effect of Arg306 → Ala and Arg506 → Gln substitutions in the inactivation of recombinant human factor Va by activated protein C and protein S. Protein Science 1997;6:2016-27.
Esmon CT. The roles of protein C and thrombomodulin in the regulation of blood coagulation. J.Biol.Chem. 1989;264:4743-4746
Gruber A, Griffin JH. Direct Detection of Activated Protein C in Blood From Human Subjects. Blood 1992; 79: 2340-48
Jenny RJ, Pittman DD, Toole JJ, Kriz RW, Aldape RA, Hewick RM, Kaufman RJ, Mann KG. 1987 Proc. Natl. Acad. Sci. USA 84: 4846-50. Complete cDNA and derived amino acid sequence of human factor V.
Mann KG, Kalafatis M. 2003. Blood 101 (1): 20-30. Factor V: a combination of Dr Jekyll and Mr Hyde.
Meijers JC, Davie EW, Chung DW. 1992. Blood 79: 1435-1440. Expression of human blood coagulation factor XI: characterization of the defect in factor XI type III deficiency.
O'connell NM. 2004. Semin Hematol. 41 (1 Suppl 1): 76-81. Factor XI deficiency.
Pittman DD, Marquette KA and Kaufman RJ (1994). Role of the B domain for Factor VIII and Factor V expression and function. Blood, 84, 4214-4225.
Salomon O, Zivelin A, Livnat T, Seligsohn U. 2006. Semin. Hematol. 43 (1 Suppl 1: S10-12. Inhibitors to Factor XI in patients with severe Factor XI deficiency.
Schulman S, Németh G. 2006. Haemophilia 12: 223-227. An illustrative case and a review on the dosing of recombinant factor VIIa in congenital factor XI deficiency.
Svensson PJ, Dahlback B. Resistance to activated protein C as a basis for venous thrombosis. NEJM 1994;330:517-522
Thorelli E, Kaufman RJ, Dahlback B. Cleavage of factor V at Arg 506 by activated protein C and the expression of anticoagulant activity of factor V Blood 1999;93:2552-58
Van Dijk K, van der Bom JG, Fischer K, Grobbee DE, van der Berg HM. 2004. Do prothrombotic factors influence clinical phenotype of severe haemophilia? A review of the literature. Thrombosis Haemostasis 92, 305-310
Van 't Veer C, Golden NJ, Kalafatis M, Simioni P, Bertina RM, Mann KG. An In Vitro Analysis of the Combination of Hemophilia A and Factor VLEIDEN. Blood 1997;90:3067-72
Williamson D, Brown K, Luddington R, Baglin C, Baglin T. 1998 Blood 91 (4): 1140-44. Factor V Cambridge: a new mutation (Arg306-->Thr) associated with resistance to activated protein C.

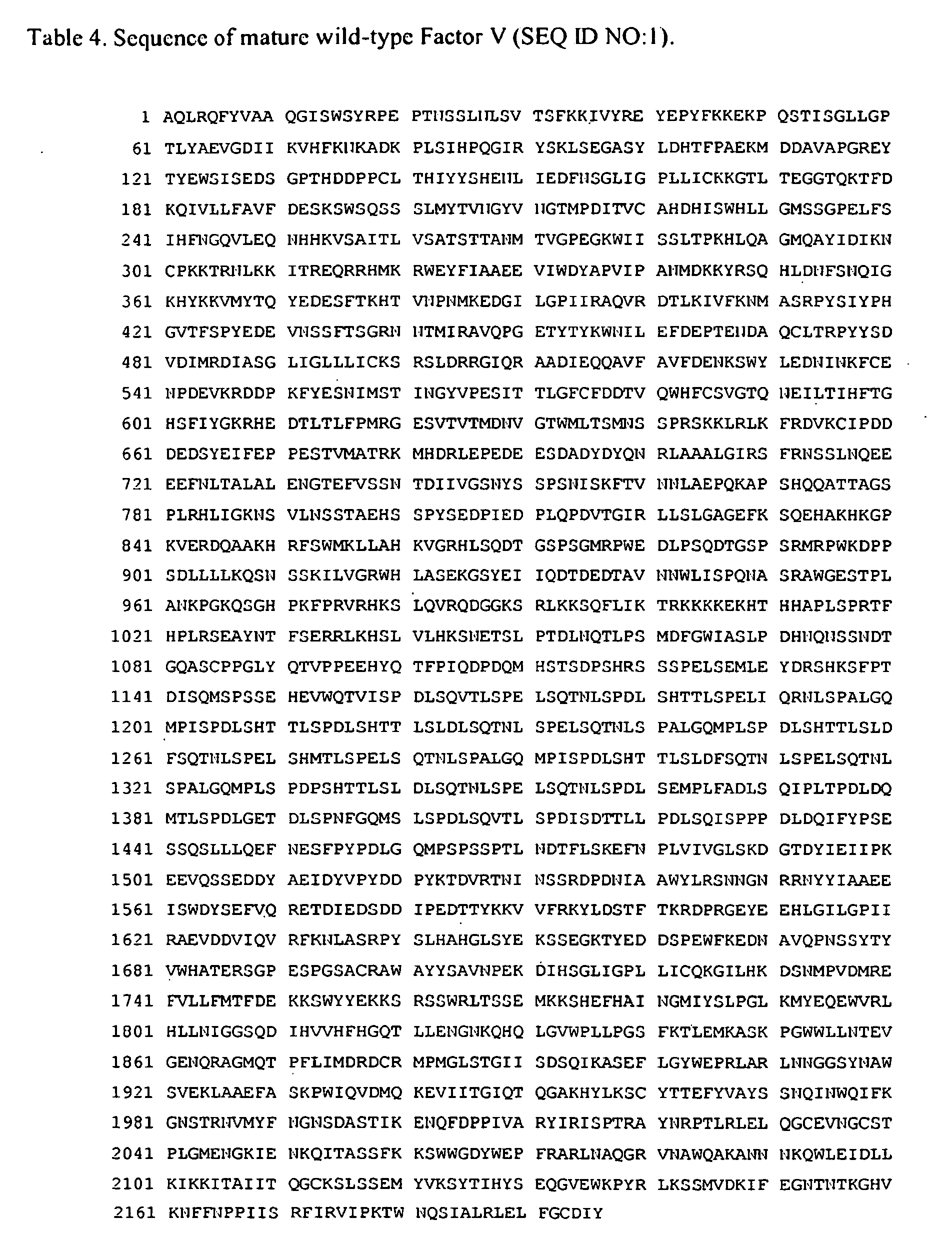

## Claims

1. APC-resistant (activated protein C resistant) Factor V for use in the prevention or treatment of bleeding in a patient with a Factor XI-deficiency.

2. APC-resistant Factor V for use in reducing or preventing the possibility of generating inhibitors to Factor XI in a hemophilia C patient.

3. APC-resistant Factor V for use according to any one of the preceding claims, wherein the APC-resistant Factor V has a mutation of Arg306, Arg506 or both Arg 306 and Arg506 as compared to the wild type Factor V sequence.

4. APC-resistant Factor V for use according to claim 3, wherein the APC-resistant Factor V has a mutation of Arg306 and Arg506 as.compared to the wild type Factor V sequence.

5. APC-resistant Factor V for use according to any one of the preceding claims, wherein the APC-resistant Factor V is free from other clotting factors.

6. APC-resistant Factor V for use according to any one of the preceding claims, wherein APC-resistant Factor V is administered to obtain a plasma concentration thereof of between 0.1 and 5 Units/ml.

7. APC-resistant Factor V for use according to any one of the preceding claims, wherein the APC-resistant Factor V has been obtained by recombinant expression.

## Patentansprüche

1. APC-resistenter (aktiviertes Protein C-resistenter) Faktor V zur Verwendung in der Vorbeugung oder Behandlung von Blutungen in einem Patienten mit einem Faktor XI-Mangel.

2. APC-resistenter Faktor V zur Verwendung in der Verminderung oder Vorbeugung der Möglichkeit der Erzeugung von Inhibitoren gegen den Faktor XI in einem Hämophilie-C-Patienten.

3. APC-resistenter Faktor V zur Verwendung nach einem der vorhergehenden Ansprüche, wobei der APC-resistente Faktor V eine Mutation von Arg306, Arg506 oder beiden Arg306 und Arg506 verglichen mit der Wildtyp-Faktor V-Sequenz hat.

4. APC-resistenter Faktor V zur Verwendung nach Anspruch 3, wobei der APC-resistente Faktor V eine Mutation von Arg306 und Arg506 verglichen mit der Wildtyp-Faktor V-Sequenz hat.

5. APC-resistenter Faktor V zur Verwendung nach einem der vorhergehenden Ansprüche, wobei der APC-resistente Faktor V frei von anderen Gerinnungsfaktoren ist.

6. APC-resistenter Faktor V zur Verwendung nach einem der vorhergehenden Ansprüche, wobei der APC-resistente Faktor V verabreicht wird, um eine Plasmakonzentration davon zwischen 0,1 und 5 Einheiten/ml zu erhalten.

7. APC-resistenter Faktor V zur Verwendung nach einem der vorhergehenden Ansprüche, wobei der APC-resistente Faktor V durch rekombinante Expression erhalten wurde.

## Revendications

1. Facteur V résistant à l'APC (résistant à la protéine C activée) à utiliser dans la prévention ou dans le traitement du saignement chez un patient présentant une déficience en facteur XI.

2. Facteur V résistant à l'APC à utiliser dans la réduction ou la prévention du risque de générer des inhibiteurs du facteur XI dans un patient souffrant d'hémophilie C.

3. Facteur V résistant à l'APC à utiliser conformément à l'une quelconque des revendications précédentes, dans lequel le facteur V résistant à l'APC possède une mutation de Arg306, de Arg506, ou à la fois de Arg306 et de Arg506 par comparaison à la séquence du facteur V de type sauvage.

4. Facteur V résistant à l'APC à utiliser conformément à la revendication 3, dans lequel le facteur V résistant à l'APC possède une mutation de Arg306 et de Arg506 par comparaison à la séquence du facteur V de type sauvage.

5. Facteur V résistant à l'APC à utiliser conformément à l'une quelconque des revendications précédentes, dans lequel le facteur V résistant à l'APC est exempt d'autres facteurs de coagulation.

6. Facteur V résistant à l'APC à utiliser conformément à l'une quelconque des revendications précédentes, dans lequel le facteur V résistant à l'APC est administré pour obtenir une concentration plasmatique dudit facteur entre 0,1 et 5 unités/ml.

7. Facteur V résistant à l'APC à utiliser conformément à l'une quelconque des revendications précédentes, dans lequel le facteur V résistant à l'APC est obtenu par expression recombinante.
